# EUROPÄISCHE PATENTSCHRIFT

(11) **EP 3 011 338 B1**
(45) Veröffentlichungstag und Bekanntmachung des Hinweises auf die Patenterteilung: **21.11.2018**
(21) Anmeldenummer: 14739069.4
(22) Anmeldetag: 23.06.2014
(51) Int. Cl.: G01N 33/569

(54) **SCHNELLTEST ZUM NACHWEISEN VON ERREGERMATERIAL, INSBESONDERE ZUR UNTERSTÜTZUNG DER DIAGNOSE EINER SEPSIS, SOWIE KIT UND VORRICHTUNG ZUR DURCHFÜHRUNG EINES SEPSISTESTS**
RAPID TEST FOR DETECTING PATHOGEN MATERIAL, IN PARTICULAR IN ORDER TO SUPPORT THE DIAGNOSIS OF SEPSIS, AND KIT AND DEVICE FOR PERFORMING A SEPSIS TEST
TEST RAPIDE POUR DÉPISTER UN MATÉRIAU PATHOGÈNE, EN PARTICULIER POUR AIDER AU DIAGNOSTIC D'UNE SEPTICÉMIE, AINSI QUE TROUSSE ET DISPOSITIF POUR EFFECTUER UN TEST DE SEPTICÉMIE

(30) Priorität: 21.06.2013 DE 102013211850
(43) Veröffentlichungstag der Anmeldung: 27.04.2016
(73) Patentinhaber: GILUPI GmbH, 14473 Potsdam (DE)
(72) Erfinder: BOLLMAN, Andreas, 12685 Berlin (DE); NIESTROJ, Robert, 10269 Berlin (DE); LÜCKE, Klaus, 14542 Werder (Havel) (DE); KRUSEKOPF, Solveigh, 13158 Berlin (DE)
(74) Vertreter: Grünecker Patent- und Rechtsanwälte PartG mbB
(86) Internationale Anmeldenummer: PCT/EP2014/063172
(87) Internationale Veröffentlichungsnummer: WO 2014/202785

(56) Entgegenhaltungen:
- WO-A1-2004/014423
- WO-A1-2005/046713
- WO-A1-2007/065423
- WO-A1-2009/036081
- WO-A2-01/94543
- WO-A2-2009/104075
- US-A1- 2010 021 942
- PISAKE BOONTHAM ET AL: "Significant immunomodulatory effects of Pseudomonas aeruginosa quorum-sensing signal molecules: possible link in human sepsis", CLINICAL SCIENCE, Bd. 115, Nr. 11, 1. Dezember 2008 (2008-12-01), Seite 343, XP055161073, ISSN: 0143-5221, DOI: 10.1042/CS20080018
- RAJAMANI SATHISH ET AL: "A LuxP-FRET-Based Reporter for the Detection and Quantification of AI-2 Bacterial Quorum-Sensing Signal Compounds", BIOCHEMISTRY, AMERICAN CHEMICAL SOCIETY, US, Bd. 46, Nr. 13, 3. April 2007 (2007-04-03) , Seiten 3990-3997, XP002429872, ISSN: 0006-2960, DOI: 10.1021/BI602479E
- WIKIPEDIA: "Autoinducer", INTERNET CITATION, 17. März 2013 (2013-03-17), Seiten 1-7, XP007922952, Gefunden im Internet: URL:https://web.archive.org/web/2013042717 0455/http://en.wikipedia.org/wiki/Autoindu cer [gefunden am 2015-01-12]

## Beschreibung

Die vorliegende Erfindung betrifft einen Schnelltest zum Nachweisen von Erregermaterial, insbesondere zur Unterstützung der Diagnose einer Sepsis, umfassend die Schritte des in Kontaktbringens einer Probe und wenigstens eines Detektionsmoleküls unter Bedingungen, die ein Binden eines Zielmoleküls aus der Probe an das wenigstens eine Detektionsmolekül ermöglichen, sowie den weiteren Schritt des des Überprüfens, ob ein Zielmolekül an das wenigstens eine Detektionsmolekül gebunden hat. Die vorliegende Erfindung betrifft ferner Kit und eine Vorrichtung zur Durchführung eines Sepsistests.

Diagnosekriterien für Sepsis und schwerer Sepsis entsprechend den ACCP/SCCM Konsensus-Konferenz Kriterien. Dies beinhaltet (1.) den Nachweis der Infektion über den mikro. Nachweis oder durch klinische Kriterien; (2.) Bestimmung der Severe Inflammatory Host Response (SIRS), bei der mindestens 2 der folgenden Kriterien erfüllt sein müssen:
- Fieber *(*≥ 38°*C)* oder Hypothermie *(*≤ 36°*C)*
- Herzrasen (Tachykardie): Herzfrequenz ≥ 90/min.
- Überhöhte Atemfrequenz (Tachypnoe) oder Hyperventilation.
- Erhöhung der Leukozyten (Leukozytose) oder
- Abnahme der Leukozyten (Leukopenie);
sowie (3.) Feststellen einer Akute Organdysfunktion mit mindestens einem der folgenden Kriterien:
- Akute krankhafte Veränderung des Gehirns (Enzephalopathie): eingeschränkte Vigilanz, Desorientiertheit, Unruhe, Delirium.
- Thrombozytopenie: Abfall der Thrombozyten um mehr als 30 % innerhalb von 24 Stunden oder Thrombozytenzahl ≤ 100.000/mm³.
- Verringerter Sauerstoffgehalt im Blut (Arterielle Hypoxämie)
- Nierenversagen (Renale Dysfunktion)
- Übersäuerung des Blutes (Metabolische Azidose).

Im Verlauf einer Sepsis kommt es häufig zu lebensbedrohlichen Störungen der Vitalfunktion und zum Versagen eines oder mehrerer Organe und letztendlich zum Tod des Patienten. Die Intensivmedizin kann zwar durch vorübergehenden Einsatz oder Unterstützung der Organfunktionen kritische Phasen überbrücken. Dies ist jedoch nur dann möglich, wenn das Auftreten der Sepsis rechtzeitig erkannt wird. Zeigen sich bereits äußerlich erkennbare Sepsissymptome, wie beispielsweise eine erhöhte Körpertemperatur, Herzfrequenz oder Atemfrequenz, kann es schon zu spät für eine erfolgreiche Behandlung sein.

Sobald äußerliche Symptome auftreten und ein Verdacht auf Sepsis besteht, werden Untersuchungen angestellt, um die Erreger beispielsweise in Blutkultur nachzuweisen. Der Nachweis des Erregers in Blutkultur dauert jedoch mindestens 24, eher 48 Stunden. Solange kann bei einem Verdacht auf Sepsis nicht gewartet werden, so dass gerade bei Intensivpatienten präventiv und nicht zielgerichtet Breitbandantibiotika verabreicht werden, in der Hoffnung, dass diese beim Erreger wirksam sind.

Es gibt ferner Biomarkeruntersuchungen, mit denen zur Früherkennung einer Sepsis Procalcitonin bestimmt wird. Mit der Bestimmung von Procalcitonin kann aber lediglich ein erhöhtes Risiko des Vorliegens einer generellen Infektion bestätigt werden. Ein solcher Test ersetzt dagegen nicht einen spezifischen Erregernachweis und ist auch nicht immer in der Lage, eine Sepsisdiagnose zu stützen, weil eine Sepsis je nach Erreger auch bei normalen, unauffälligen Konzentrationen von Procalcitonin vorliegen kann.

Die WO 2005/046713 A1 betrifft ein Verfahren zur Regulierung von Quorum Sensing in Bakterien mittels Modulation der Aktivierung von LuxR durch ein Signalmolekül, beispielsweise N-Acyl-Homoserinlacton. Ferner offenbart die WO 2005/046713 A1 ein Verfahren zur Detektion von Quorum-Sensing-Bakterien unter Verwendung eines Antikörpers gegen LuxR und nennt die Verwendung eines solchen Antikörpers bei der Behandlung von Erkrankungen, die durch Quorum Sensing ausgelöst werden. Schließlich sind in der WO 2005/046713 A1 Nachweistests, beispielsweise ein ELISA-Test für den Nachweis von Bakterien, die Quorum Sensing nutzen, beschrieben, die auch in der Diagnostik eingesetzt werden können.

Die WO 2009/036081 A1 betrifft ein Verfahren zur Diagnose von einer bakteriellen Infektion durch Bestimmen der Menge wenigstens eines Quorum Sensing Moleküls in einer Probe und Vergleichen der bestimmten Menge mit einem Kontrollwert. Das Quorum-Sensing-Molekül kann beispielsweise N-Acylhomoserinlacton oder Autoinducer-2 sein. Für die Bestimmung der Menge des Quorum-Sensing-Moleküls wird bevorzugt ein Zellerkennungssystem verwendet.

In der WO 2007/065423 A1 ist ein Verfahren sowie eine Vorrichtung zur Detektion von Mikroorganismen und/oder deren Aktivität mittels auf Quorum-Sensing-Komponenten basierender Biosensoren beschrieben. Dazu wird an einer Oberfläche eines Substrates bereichsweise wenigstens ein Ligand und/oder Rezeptor immobilisiert, wobei durch Bindungen von Stoffen, die von Mikroorganismen im Prozess des Quorum-Sensing ausgesandt werden, hervorgerufene physikalische oder physikalisch-chemische Änderungen messbar sind. Die Gruppe von Autoinducern aus acylierten Homoserin-Laktonen bzw. als Rezeptoren agierende Proteine des LuxP-Typs werden als immobiliserte Fängermoleküle genannt.

In der US 2010/021942 A1 ist ein zellfreies Expressionssystem zur Detektion eines bakteriellen Biofilms auf einer Oberfläche beschrieben. Das System umfasst ein exogenes Quorum-Sensing-Protein, das selektiv ein bakterielles Quorum-Sensing-Molekül bindet, beziehungsweise eine ein solches Protein kodierende Nukleinsäure, sowie eine exogene Nukleinsäure umfassend einen Promotor und eine Nukleinsäuresequenz, die ein Markerprotein kodiert, wobei der Promotor über die Bindung des Quorum-Sensing-Proteins mit dem Signalmolekül reguliert wird. Beispielhafte bakterielle Signalmoleküle sind Homoserinlaktone oder Autoinducer, beispielsweise Furasonylboratdiester.

Die WO 2009/104075 A2 betrifft einen Sandwich-Assay mit einem Fängerkonjugat, das aus einem zielspezifischen DNA-Aptamer, gebunden an einem magnetischen Kügelchen, sowie einem Reporterkonjugat, bestehend aus einem DNA-Aptamer und einer Nachweissubstanz besteht. Die beiden DNA-Aptamere binden ein Analyt, beispielsweise ein pathogenes Bakterium oder ein Quorum-Sensing-Molekül wie beispielsweise Acylhomoserinlaktone an unterschiedlichen Epitopen.

Die WO 01/94543 A2 befasst sich mit Autoinducer-analogen Molekülen, die so derivatisiert sind, dass sie eine reaktive Gruppe aufweisen, die eine Anbindung der analogen Moleküle an Oberflächen oder anderen Molekülen erlaubt. Als Einsatzgebiete für derartige analoge Moleküle werden Assays, beispielsweise ELISA-Assays genannt.

In der WO 2004/014423 A1 sind Antikörper gegen Homoserinlactone bzw. Furasonylboratdiester sowie ein Einsatz dieser Antikörper zur Behandlung einer bakteriellen Infektion beschrieben.

Die Veröffentlichung von Boontham et al. ("Significant immunomodulatory effects of Pseudomonas aeruginosa quorum-sensing signal molecules: possible link in human sepsis", CLIN-ICAL SCIENCE, Bd. 115, Nr. 11,1. Dezember 2008 (2008-12-01), Seite 343) befasst sich mit einem möglichen Zusammenhang zwischen immunmodulierenden Effekten von Quorum-Sensing-Molekülen aus Pseudomonas aerogenosa und humaner Sepsis. Diese These wird deshalb aufgestellt, weil in Patienten mit Sepsis immunologische Veränderungen festgestellt wurden, deren Ursache in den Auswirkungen von Quorum-Sensing-Molekülen auf Immunzellen vermutet werden. So stellten die Autoren fest, dass AHL-Biosensoren in Patienten mit septischen Schock in 86 % der Fälle aktiviert waren, wohingegen der Biosensor bei gesunden Patienten nicht aktiviert wurde.

In Rajamani et al. ("A LuxP-FRET-Based Reporter for the Detection and Quantification of Al-2 Bacterial Quorum-Sensing Signal Compounds", BIOCHEMISTRY, AMERICAN CHEMI-CAL SOCIETY, US, Bd. 46, Nr. 13, 3. April 2007 (2007-04-03), Seiten 3990-3997) ist ein in vitro Assay und Sensor für die schnelle Detektion und Quantifizierung von Furasonylboratdiester (Autoinducer 2) beschrieben. Der Sensor basiert auf einer Änderung des Fluoreszenzresonanzenergietransfers zwischen zwei Varianten von GFPs, der bei Bindung von Autoinducer 2 am Lux P hervorgerufen wird. Damit sei ein schneller Nachweis von Autoinducer 2 in fünf Minuten möglich.

Der vorliegenden Erfindung liegt somit die Aufgabe zugrunde, einen Schnelltest und eine Vorrichtung bereit zu stellen, welche es ermöglicht, einen Erreger für Sepsis frühzeitig zu erkennen, Sepsis-Erreger zu detektieren, deren pathogenen Status zu ermitteln bzw. den verursachenden Keim näher zu charakterisieren.

Die vorliegenden Erfindung löst diese Aufgabe durch ein Schnelltestverfahren zum Nachweis von Erregermaterial umfassend die Schritte:
- in Kontaktbringen einer Probe und wenigstens zweier Detektionsmoleküle, die ein unterschiedliches Quorum Sensing-Zielmolekül binden, unter Bedingungen, die ein Binden der Zielmoleküle an die wenigstens zwei Detektionsmoleküle ermöglichen, wobei die unterschiedlichen Quorum Sensing Zielmoleküle ein Homoserinlacton und ein Autoinduktor-Peptid sind; und
- Überprüfen, ob Zielmoleküle an die wenigstens zwei Detektionsmoleküle gebunden haben.

Die erfindungsgemäße Vorrichtung löst die Aufgabe dadurch, dass sie wenigstens eine Vorrichtung zur Durchführung eines Sepsistestes, insbesondere in Form eines Schnelltestverfahrens, die wenigstens zwei Nachweisfelder mit Detektionsmolekülen, die ein Quorum Sensing-Zielmolekül spezifisch binden, umfasst, wobei das Quorum Sensing Zielmolekül ein Homoserinlacton oder ein Autoinduktor-Peptid ist, und wobei die Detektionsmoleküle in einem Nachweisfeld ein Homoserinlacton und in einem anderen Nachweisfeld ein Autoinduktor-Peptid spezifisch binden.

Das erfindungsgemäße Kit löst die Aufgabe dadurch, dass es wenigstens Kit zur Durchführung eines Sepsis-Testes, insbesondere in Form eines Schnelltestverfahrens, umfassend wenigstens zwei Detektionsmoleküle, die ein unterschiedliches Quorum Sensing-Zielmolekül spezifisch binden, wobei die unterschiedlichen Quorum Sensing Zielmoleküle ein Homoserinlacton und ein Autoinduktor-Peptid sind, sowie ferner umfassend wenigstens einen der folgenden Teile:
- ein Puffer,
- ein Waschreagenz,
- ein Nachweisreagenz zum Ausgeben eines messbaren Signals.

Die Erfinder haben überraschenderweise herausgefunden, dass eine erhöhte Konzentration an Quorum Sensing Molekülen bzw. Quorum Sensing-assoziierter Moleküle ein starkes Indiz für eine Sepsisgefahr darstellt und man mittels Detektion von Quorum Sensing Zielmolekülen bzw. Quorum Sensing-assoziierten Zielmolekülen die Erreger einer Sepsis und deren pathogenen Status ermitteln kann. Die Erfindung ermöglicht es, schneller und spezifischer als bisher eine Sepsisdiagnose zu stützen und deren Erreger näher zu charakterisieren. Nachweisbares Quorum Sensing zusammen mit z. B. Fieber und Blutdruckabfall ist viel spezifischer als Fieber, Blutdruck und irgendein Entzündungsparameter. Dies erlaubt es behandelnden Ärzten einer Intensivstation, aber auch in der Ambulanz zielgerichtete Maßnahmen für die Behandlung der Sepsis einzuleiten, wodurch der präventive Einsatz von Breitbandantibiotika gegen sämtliche möglichen Sepsis-Erreger vermieden wird. Auf diese Weise kann die Aussagekraft des Schnelltests, Kits bzw. der erfindungsgemäßen Vorrichtung verbessert werden, indem mehr als ein Zielmolekül, dessen Vorhandensein in Indiz für Sepsis ist bzw. das für einen speziellen Erreger charakteristisch ist, nachgewiesen werden kann. Der erfindungsgemäße Schnelltest und das erfindungsgemäße Verfahren ermöglichen es insbesondere, die Infektion nachzuweisen, indem u. a. Gram-Status und die Pathogenität des Erregers bestimmt werden können.

Die erfindungsgemäße Vorrichtung ist besonders gut geeignet als patientennahe Labordiagnostik (Point-Of-Care-Testing), also zur Unterstützung diagnostischer Untersuchungen, die nicht in einem Zentrallabor, sondern in unmittelbarer Nähe zum Patienten, beispielsweise auf der Intensivstation des Krankenhauses, in Ambulanzen oder in Notfallsituationen in der Wohnung eines Patienten oder in einem Notarztwagen vorgenommen werden können.

Ein Schnelltest umfasst Untersuchungen, die in unmittelbarer Nähe zum Patienten durchgeführt werden können und deren Ergebnis in höchstens zwei Stunden, vorzugsweise in weniger als einer Stunde, bevorzugt innerhalb von bis zu 15 Minuten vorliegt.

Unter einer spezifischen Bindung im Sinne der vorliegenden Erfindung wird eine Bindung verstanden, deren Affinität so hoch ist, dass sie eine Assoziationskonstante (auch Bindungskonstante genannt) von mindestens 10⁴ Mol⁻¹, vorzugsweise von mindestens 10⁵ Mol⁻¹ und insbesondere von über 10⁶ Mol⁻¹ aufweist.

Quorum Sensing Zielmoleküle sind Moleküle, mit denen Bakterien, Hefen und Pilzen kommunizieren. Quorum Sensing-assoziierte Zielmoleküle sind Stoffe, die infolge einer erhöhten Konzentration von Quorum Sensing Molekülen verstärkt produziert werden und/oder in ihrer Funktion bzw. Struktur, im Vergleich zu einer Umgebung ohne bzw. einer geringen Konzentration an Quorum Sensing Molekülen, verändert sind.

Das Überprüfen, ob ein Zielmolekül an das wenigstens eine Detektionsmolekül gebunden hat, kann auf verschiedenste Weise erfolgen, beispielsweise colormetrisch, elektrisch oder chemisch, worauf nachfolgend noch näher eingegangen wird. Wird im Rahmen des Überprüfens festgestellt, dass ein Quorum Sensing Zielmolekül und/oder Quorum Sensing-assoziiertes Zielmolekül an das wenigstens eine Detektionsmolekül gebunden hat, also in der Probe enthalten war, liegt eine Sepsis vor.

Die vorliegende Erfindung kann durch eine Reihe von voneinander unabhängigen, jeweils für sich vorteilhaften und beliebig miteinander kombinierbaren Weiterbildung, wie sie im Folgenden beschreiben sind, weiter verbessert werden.

Gemäß einer ersten Ausführungsform kann das erfindungsgemäße Kit eine erfindungsgemäßen Vorrichtung umfassen, bzw. bei einem erfindungsgemäßen Schnelltest die Probe mit einer erfindungsgemäßen Vorrichtung in Kontakt gebracht werden.

Gemäß einer weiteren vorteilhaften Ausgestaltung kann das Detektionsmolekül ein Antikörper, ein spezifisch bindendes Fragment von einem Antikörper, ein Antigen, ein Peptid, ein Protein, eine Nukleinsäure, ein Aptamer, ein Ligand, ein Rezeptor, ein Hapten, ein Enzym, ein Enzyminhibitor, ein Enzymsubstrat oder ein Cofaktor von einem Enzym sein. Die Verwendung derartiger Detektionsmoleküle in der erfindungsgemäßen Vorrichtung/Kit bzw. dem erfindungsgemäßen Schnelltest ermöglicht ein Binden und Nachweisen der Zielmoleküle mit der erforderlichen spezifischen Affinität. Bei Verwendung solcher Detektionsmoleküle ist es zudem möglich, anhand etablierter Assays, beispielsweise eines Immunassays oder eines Enzymassays bzw. einer enzymatischen Analyse zu überprüfen, ob ein Zielmolekül an das wenigstens eine Detektionsmolekül gebunden hat.

Als Probe, die mittels des erfindungsgemäßen Schnelltests auf das Vorliegen einer Sepsis hin überprüft wird, kann eine Blutprobe, eine Urinprobe, eine Sputumprobe, eine Lymphflüssigkeitsprobe oder eine sonstige Körperflüssigkeitsprobe verwendet werden. Die Probe kann entweder direkt, also ohne Probenvorbereitung auf die erfindungsgemäße Vorrichtung gegeben bzw. in dem erfindungsgemäßen Schnelltest mit Detektionsmolekülen in Kontakt gebracht werden. Probenvorbereitungen, beispielsweise Filtrieren oder Ausfällen von unerwünschten Probenbestandteilen, oder ein Aufschluss von Probenmaterial sind erfindungsgemäß ebenfalls möglich und können im entsprechenden Umfeld das Ergebnis der erfindungsgemäßen Vorrichtung und Kit bzw. des erfindungsgemäßen Schnelltests positiv beeinflussen.

In einer weiteren Ausführungsform kann das erfindungsgemäße Verfahren zwischen dem in Kontakt bringen und dem Überprüfen einer Bindung einen Zwischenschritt umfassen, bei dem nicht gebundenes Probenmaterial entfernt wird. Zu diesem Zweck können die Detektionsmoleküle direkt oder indirekt über ein Verknüpfungsmolekül und/oder eine Polymermatrix auf einem Träger immobilisiert sein. Als Trägermaterial kommen beispielsweise ein Teststreifen, ein Teststäbchen, eine Mikrotiterplatte, ein Biochip, ein Mikroarraysubstrat, aber auch ein Katheter, eine Kanüle oder ein Signalwandler in Betracht. Eine Vorrichtung mit immobilisierten Detektionsmolekülen kann, nachdem die Probe appliziert wurde, mit einer Waschlösung behandelt und ungebundenes Probenmaterial abgewaschen werden, bevor überprüft wird, ob ein Zielmolekül an das wenigstens eine Detektionsmolekül gebunden hat. Teststreifen und Teststäbchen haben den Vorteil, dass die Untersuchung, nach Applikation der Probe automatisch ablaufen kann, weil die Probenflüssigkeit aufgrund der Kapillarkräfte in dem Trägerstreifen bzw. -stäbchen, beispielsweise einer Nitrocellulosemembran, zu den entsprechenden Reagenzien und Nachweisfeldern transportiert wird. Eine Mikrotiterplatte, ein Biochip und ein Mikroarraysubstrat als Träger haben den Vorteil, dass die erfindungsgemäße Vorrichtung kompakt ist und gleichwohl mit einer Vielzahl an Nachweisfeldern bestückt werden kann. Beispielsweise kann jede der Vertiefungen eine Mikrotiterplatte ein separates Nachweisfeld ausbilden.

Wird ein Katheter oder eine Kanüle als Träger der erfindungsgemäßen Vorrichtung eingesetzt, kann der Test besonders schnell, quasi in Echtzeit, durchgeführt werden, weil in diesem Fall eine Blutprobe beim bestimmungsgemäßen Gebrauch der Kanüle bzw. des Katheters zwangsläufig mit den Detektionsmolekülen auf dem Träger in Kontakt gebracht wird.

Das Überprüfen einer Bindung zwischen Detektionsmolekül und Zielmolekül kann erleichtert werden, indem die Detektionsmoleküle auf einem Signalwandler, beispielsweise einem Signalwandler eines Biosensors immobilisiert sind. Der Signalwandler setzt die Bindung zwischen Detektionsmolekül und Zielmolekül in ein Bindungssignal um, das anschließend beispielsweise über einen Signalleiter, der kabelgebunden oder kabelungebunden sein kann, übertragen und von einer entsprechenden Messvorrichtung ausgelesen werden kann. Als Träger kommt beispielsweise ein elektrochemischer Signalwandler, ein optischer Signalwandler, ein akustischer Signalwandler, ein elektrischer Signalwandler, ein thermischer Signalwandler, ein piezoelektrischer Signalwandler oder ein biochemischer Signalwandler in Betracht. Gut geeignet sind elektrochemische und/oder optische Signalwandler, die robust sind und eine zuverlässige Umwandlung der Bindung eines Zielmoleküls mit einem Detektionsmolekül in ein messbares Signal ermöglichen. Ein elektrochemischer Signalwandler kann die Bindung beispielsweise in eine Änderung des Widerstandes, der Impedanz oder des Stromflusses umwandeln. Ein optischer Signalwandler kann eine Änderung der Lichtbrechung, wie sie bei der Oberflächenplasmonenresonanzspektroskopie erfolgt, als Bindungssignal ausgeben. Als biochemischer Signalwandler kann beispielsweise Histidinkinase eingesetzt werden. Histidinkinasen sind Enzyme, die eine Autophosphorylierung durchführen. Histidinkinasen können mit einem Detektionsmolekül derart gekoppelt werden, dass die Histidinkinase erst nach dem Binden eines Zielmoleküls an das gekoppelte Detektionsmolekül aktiviert wird. Die Aktivierung setzt eine Autophosphorylierung in Gang, bei der ATP in ADP umgesetzt werden kann. Der ATP-Umsatz wiederum kann als Bindungssignal ausgegeben und beispielsweise colormetrisch sichtbar gemacht oder durch Elektronenübergang auf einem Halbleiter in ein elektrisches Bindungssignal überführt werden.

Gemäß einer weiteren Ausführungsform einer erfindungsgemäßen Vorrichtung mit einem Katheter als Trägermaterial kann die erfindungsgemäße Vorrichtung ferner einen Signalwandler, der die Bindung eines Zielmoleküls an dem wenigstens einen mit Detektionsmolekülen versehenen Nachweisfeld in ein Bindungssignal umwandeln, sowie einen Signalleiter zur Übertragung des Bindungssignals umfassen. Auf diese Weise ist es möglich, das Sepsis spezifische bzw. für einen Erregertyp charakteristische Zielmolekül in Echtzeit nachzuweisen, wenn der Katheter dieser Ausführungsform bestimmungsgemäß eingesetzt wird, beispielsweise als Herzkatheter oder Blasenkatheter, weil er mit sämtlichen Bestandteilen eines herkömmlichen Biosensors ausgerüstet ist, welcher die Umwandlungsübertragung eines Signals der Bindung ermöglichen.

Wie oben bereits erwähnt, können die Detektionsmoleküle direkt oder indirekt über ein Verknüpfungsmolekül (auch Linker genannt) und/oder eine Polymermatrix auf einem Träger immobilisiert sein. Eine Poymermatrix ist aufgrund der vielseitigen Eigenschaften gut für die Modifizierung der Oberfläche des Trägers einsetzbar und die Vielzahl verschiedener Polymere und Möglichkeiten zur Modifikation dieser Polymere erlauben eine speziell für den jeweiligen Einsatzzweck der erfindungsgemäßen Vorrichtung geeignete Polymermatrix auszuwählen. Gut eignen sich beispielsweise eine Polymermatrix, die funktionelle Gruppen aufweist, über welche die Detektionsmoleküle direkt angebunden werden können. Weitere vorteilhafte Eigenschaften einer Polymermatrix sind nachfolgend aufgelistet:
- Die Polymermatrix ist als zusammenhängende Polymerschicht ausgebildet. Dadurch kann die gesamte Oberfläche des Trägers durch die Polymermatrix abgedeckt oder abgeschirmt werden. Die Dicke der Polymerschicht liegt vorzugsweise im Bereich von 0,1 bis 10 µm, bevorzugt im Bereich von 0,5 bis 5 µm, besonders bevorzugt im Bereich von 1 bis 2 µm.
- Die Polymermatrix weist eine dreidimensionale, vorzugsweise filamentöse und/oder poröse Struktur auf. Die Polymermatrix weist eine kohlenstoffhaltige, verzweigte Molekülstruktur auf. Diese Strukturen eignen sich hervorragend für die Anbindung der Detektionsmoleküle und vergrößern die effektive Bindungsfläche. Im Bereich der Grenzschicht an einer mit dieser Molekülstruktur besetzten Oberfläche wird die Umströmung von einer Probenflüssigkeit erheblich verlangsamt. Dadurch wird die Anreicherung der Liganden begünstigt. Mögliche vorteilhafte Ausgestaltungen der strukturierten Funktionsfläche sind eine Funktionsfläche mit Erhöhungen, Vertiefungen und/oder Verzweigungen, und/oder eine Funktionsfläche, die zumindest teilweise eine spiralförmige, schraubenförmige, schneckenförmige, wellenförmige, helikale, filamentöse, bürstenförmige, kammförmige, netzförmige, poröse, schwammförmige Struktur umfasst.
- Die Polymermatrix ist vorzugsweise über funktionelle Gruppen mit dem Träger, beispielsweise dem Katheter oder Signalwandler wirkverbunden, bevorzugt durch chemische Bindung, besonders bevorzugt durch kovalente Bindung.
- Die Polymermatrix ist ein Hydrogel.
- Die Polymermatrix umfasst gesättigte Atomgruppen und kovalent gebundene Detektionsrezeptoren, um unerwünschte Wechselwirkungen mit Blutbestandteilen und die Anbindung von unspezifischen Zellen und Molekülen zu verhindern.
- Die Polymermatrix ist vernetzt.
- Die Polymermatrix umfasst bzw. bildet das Nachweisfeld. Die Polymermatrix kann direkt mit den Detektionsmolekülen bestückt sein.
- Die Polymermatrix verhindert die Anbindung unspezifischer Zellen oder Wechselwirkungen mit Körperflüssigkeiten.
- Die Polymermatrix kann Elektronen übertragen.
- Die Polymermatrix kann aus modifizierbaren Substanzen bestehen, die sich bei Bindung eines Zielmoleküls mit einem Detektionsmolekül strukturell verändern können, und auf diese Weise optische und/oder elektronische Signale induzieren.

Es ist auch möglich, die Detektionsmoleküle über Verknüpfungsmoleküle an in dem jeweiligen dafür vorgesehenen Nachweisfeld zu immobilisieren.

Gemäß einer weiteren vorteilhaften Ausführungsform des erfindungsgemäßen Schnelltests kann ein Nachweisreagenz, das ein messbares Bindungssignal ausgibt, zum Überprüfen des Bindens zugegeben werden. Das Nachweisreagenz kann beispielsweise im Falle des Bindens eine Farbreaktion oder eine chemische Reaktion, die etwa elektronisch oder über eine pH-Änderung messbar ist, in Gang setzen. Als Nachweisreagenzien werden bevorzugt Moleküle verwendet, die spezifisch den Komplex aus Detektionsmolekül und Zielmolekül binden. Ein Nachweisreagenz kann beispielsweise ein zweites, vom Detektionsmolekül nicht besetztes Epitop des Zielmoleküls binden. In Betracht kommen als Nachweisreagenzien mit einem Marker versehene Antikörper, Proteine, Aptamere oder Liganden. Der Marker kann ein messbares Bindungssignal direkt ausgeben, beispielsweise in Form eines Fluoreszenzsignals oder einer Farbgebung, oder das Bindungssignal indirekt erzeugen, beispielsweise im Rahmen einer enzymatischen Substratumsetzung einen Farbstoff oder frei Elektronen erzeugen, was colormetrisch oder über einen Halbleiter gemessen werden kann.

Gemäß einer weiteren Ausführungsform kann die erfindungsgemäße Vorrichtung eine Messvorrichtung zur Detektion wenigstens eines Zielmoleküls umfassen. Vorzugsweise umfasst die erfindungsgemäße Vorrichtung eine Messvorrichtung zur Detektion der Bindung zwischen Detektionsmolekül und Zielmolekül in jedem Nachweisfeld. Die Messvorrichtung ermöglicht einen qualitativen Nachweis, und gibt bei einer erfindungsgemäßen Umsetzung mit mehr als einer Art von Detektionsmolekül aus, welche Zielmoleküle in der Probe vorhanden sind. Eine Automatisierung des Schnelltests und die Handhabung einer erfindungsgemäßen Vorrichtung lässt sich beispielsweise dadurch verbessern, dass ein digitales Messgerät die Bindung anzeigt. Um das Zielmolekül nicht nur qualitativ nachzuweisen, sondern auch quantitativ die Menge des Zielmoleküls zu bestimmen, kann die Messvorrichtung vorzugsweise nicht nur zur Detektion, sondern auch zur Quantifizierung der Bindung zwischen Detektions- und Zielmolekül in wenigstens einem, vorzugsweise in jedem Nachweisfeld ausgestaltet sein. Der erfindungsgemäße Schnelltest kann also vorsehen, das messbare Bindungssignal zu quantifizieren. Die Quantifizierung der Menge des Zielmoleküls in der Probe hat den Vorteil, dass sich darüber Rückschlüsse über den Verlauf der Sepsis ziehen lassen. Zur Quantifizierung kann beispielsweise die Stärke des Bindungssignals herangezogen werden, die mit der Menge des Zielmoleküls, das am Detektionsmolekül gebunden hat, korrelieren kann. Die Quantifizierung kann ferner semi-quantitativ erfolgen, indem nur bei Überschreiten eines bestimmten Schwellenwertes einer Konzentration des Zielmoleküls in der Probe ein Bindungssignal äußerlich erkennbar ausgegeben wird.

Doch nicht nur der Sepsisverlauf kann mit der erfindungsgemäßen Vorrichtung, dem Kit und Schnelltest nachgewiesen werden. In Abhängigkeit vom Zielmolekül, das spezifisch mit dem Detektionsmolekül bindet, kann der Sepsis-Erreger charakterisiert, vorzugsweise der Gram-Status des Sepsis-Erregers bestimmt werden. Hierbei zahlt es sich aus, dass Quorum Sensing Zielmoleküle und/oder Quorum Sensing-assoziierte Zielmoleküle speziell nur von Gramnegativen Erregern bzw. Gram-positiven Erregern produziert werden. Beispielsweise ist Homoserinlacton ein Gram-negativ-spezifisches Quorum Sensing Zielmolekül. Quorum Sensing Oligopeptide, die als Autoinduktor-Peptide zusammengefasst werden, und zu denen beispielsweise Furanosyl-borat-diester zählt, sind spezifisch für gram-positive Erreger. Beispielhafte Homoserinlactone enthält die Gruppe der N-Acyl-Homoserinlactone (HSL) 1 bis 4 (HSL1: N-(11-carboxy-3-oxoundecanoyl)-L-homoserinlacton; HSL2: N-(5-carboxypentanoyl)-L-homoserinlacton; HSL3: N-(11-carboxy-3-hydroxyundecanoyl)-L-homoserinlacton; HSL4: N-(9-carboxynonanoyl)-L-homoserinlacton). Bei einem Schnelltest, der ein Detektionsmolekül, das spezifisch an jeweils eines der HSL 1 bis 4-Moleküle bindet bzw. mit einer Vorrichtung, die vier Nachweisfelder mit Detektionsmolekülen gegen HSL 1 bis 4 ausgerüstet ist, kann in Abhängigkeit vom Bindungsmuster sogar auf einen speziellen Erregertyp geschlossen werden.

Entsprechendes gilt für Detektionsmoleküle, die ein Quorum Sensing-assoziiertes Zielmolekül spezifisch binden. Überraschenderweise hat sich hier gezeigt, dass als direkte Folge einer erhöhten Konzentration von Quorum Sensing Molekülen die Ezymaktivitäten von beta-Galaktosidase, beta-Hexosaminidase und Arylsulphatase A erhöht und die Enzymaktivität von Paraxonase 1 (auch Paraoxonase 1) reduziert ist. Die Enzymaktivität von beta-Galaktosidase, beta-Hexosaminidase und Arylsulphatase A ist bei Sepsis, verglichen mit dem Normalwert, mindestens 4-fach, vorzugsweise mindestens 10-fach erhöht. Die Paraxonase 1 Aktivität ist bei Sepsis, verglichen mit der Aktivität ohne Sepsis, um wenigstens 33%, vorzugsweise um etwa 50% oder mehr reduziert.

Der Nachweis eines solchen Quorum Sensing-assoziierten Enzyms kann in dem erfindungsgemäßen Schnelltest beispielsweise so implementiert werden, dass das Zielmolekül ein Quorum Sensing-assoziiertes Enzym, insbesondere beta-Galaktosidase, beta-Hexosaminidase, Arylsulphatase A und/oder Paraxonase 1 ist, dessen Enzymaktivität bestimmt wird. Stellt sich bei der Bestimmung der Enzymaktivität heraus, dass diese erhöht bzw. gesenkt ist, ist dies als Nachweis einer Sepsis zu beurteilen. Gemäß einer weiteren Ausführungsform können die Detektionsmoleküle in den wenigstens zwei Nachweisfeldern spezifisch binden mit einem der Quorum Sensing Zielmoleküle N-Acyl-Homoserinlacton 1, N-Acyl-Homoserinlacton 2, N-Acyl-Homoserinlacton 3, N-Acyl-Homoserinlacton 4, oder einem Furanosyl-borat-diester. Dabei ist jede mögliche Kombination der oben genannten Quorum Sensing Zielmoleküle möglich und hiermit offenbart, auch wenn sie im Anmeldetext nicht explizit angeführt ist.

Im Folgenden wird die Erfindung anhand von Zeichnungen beispielhaft erläutert. Die unter Bezugnahme auf die Zeichnungen erläuterten Merkmalskombinationen kann jedoch nach Maßgabe der obigen Ausführungen abgeändert werden. So kann beispielsweise auf einzelne Merkmale der erfindungsgemäßen Vorrichtung verzichtet werden, wenn diese Merkmale bei einer bestimmten Anwendung keinen wesentlichen Vorteil bieten. Umgekehrt kann eines der oben beschriebenen Merkmale hinzugefügt werden, wenn der mit diesem Merkmal verbundene Vorteil für die jeweilige Anwendung vorteilhaft ist.

Es zeigen:
- Fig. 1: eine Vorrichtung in einer schematischen Darstellung;
- Fig. 2: eine schematische Darstellung verschiedener Varianten eines erfindungsgemäßen Verfahrens;
- Fig. 3: eine weitere Vorrichtung in einer schematischen Darstellung;
- Fig. 4: eine schematische Darstellung einer erfindungsgemäßen Vorrichtung gemäß einer Ausführungsform;
- Fig. 5: eine schematische perspektivische Darstellung einer weiteren Ausführungsform der erfindungsgemäßen Vorrichtung; und
- Fig. 6: eine schematische Darstellung eines Ausschnitts A aus Fig. 5.

Die Vorrichtung 1 und deren einzelne Bestandteile werden nachfolgend mit Bezug auf die beiliegenden Zeichnungen anhand beispielhafter Ausführungsformen im Detail beschrieben. Gleichzeitig wird der erfindungsgemäße Kit sowie der Schnelltest zum Nachweisen von Erregermaterial einer Sepsis unter Bezugnahme auf die in den Figuren gezeigten Ausführungsformen der Vorrichtung 1 dargelegt.

Die Vorrichtung 1, Kit sowie der erfindungsgemäße Schnelltest ermöglichen den Nachweis von Erregermaterial zur Unterstützung der Diagnose einer Sepsis auf schnellem und einfachem Wege in zuverlässiger Manier.

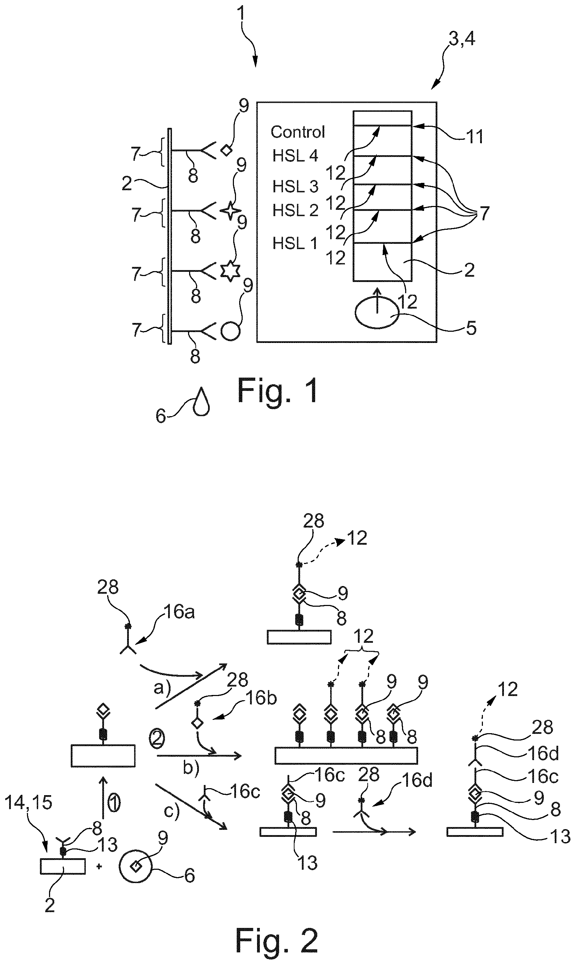

Im Folgenden wird eine Vorrichtung 1 mit Bezug auf die Fig. 1 näher erläutert. Bei der in Fig. 1 gezeigten Ausführungsform weist die Vorrichtung 1 einen Träger 2 auf, der nach Art eines Teststreifens 3 bzw. Teststäbchens 4 ausgestaltet ist. Solche Teststreifen 3 bzw. Teststäbchen 4 eignen sich besonders gut als Schnelltests für patientennahe Labordiagnostik. Die Vorrichtung 1 umfasst ein Applikationsfeld 5, auf welches eine Probe 6, in der gezeigten Ausführungsform beispielhaft eine durch einen Bluttropfen schematisch dargestellte Blutprobe, aufgetragen wird. Die Blutprobe wird aufgrund von Kapillarkräften entlang der in Fig. 1 als Pfeil dargestellten Flussrichtung im Träger 2, beispielsweise einer Nitrocellulosemembran transportiert. Während des Transports passiert die Probe 6 zunächst vier Nachweisfelder 7 und anschließend ein Kontrollfeld 11 ["*control*"].

Jedes Nachweisfeld 7 ist mit Detektionsmolekülen 8 bestückt, die ein Quorum Sensing Zielmolekül 9 und/oder ein Quorum Sensing-assoziiertes Zielmolekül 10 spezifisch binden.

In der gezeigten Vorrichtung 1 sind Antikörper als spezielle Detektionsmoleküle 8 in den vier Nachweisfeldern 7 angeordnet. Die Detektionsmoleküle 8 binden in der Fig. 1 spezifisch die Quorum Sensing Zielmoleküle 9 N-Acyl-Homoserinlacton 1 (HSL 1: dargestellt durch einen Kreis), N-Acyl-Homoserinlacton 2 (HSL 2: dargestellt durch einen sechszackigen Stern), N-Acyl-Homoserinlacton 3 (HSL 3: dargestellt durch einen vierzackigen Stern) und N-Acyl-Homoserinlacton 4 (HSL 4: dargestellt durch eine Raute).

Wird die Probe 6 mit den Detektionsmolekülen 8 in dem jeweiligen Nachweisfeld 7 in Kontakt gebracht, bindet das entsprechende Zielmolekül 9 spezifisch an den gegen ihn gerichteten Antikörper 8. In der gezeigten als Teststreifen 3 bzw. Teststäbchen 4 vorgesehenen Vorrichtung 1 sind die Detektionsmoleküle 8 direkt am Träger 2 immobilisiert, so dass die am Detektionsmolekül 8 gebundene Quorum Sensing Zielmoleküle 9 im entsprechenden Nachweisfeld 7 zurückgehalten werden, wenn die Probe 6 ein Testfeld 7 entlang ihrer Flussrichtung passiert hat.

Die in Fig. 1 gezeigte Vorrichtung umfasst somit vier Nachweisfeldern 7, wobei die Detektionsmoleküle 8 in jedem Nachweisfeld 7 ein unterschiedliches Quorum Sensing Zielmolekül 9, nämlich HSL 1 bis 4 spezifisch binden.

Das Kontrollfeld 11 kann ein Mittel 8 (nicht gezeigt) aufweisen, welches anzeigt, dass die applizierte Probe 6 vom Applikationsfeld 5 in Flussrichtung sämtliche Nachweisfelder 7 passiert hat und bis in das Kontrollfeld 11 geflossen ist. In dem Kontrollfeld 11 kann beispielsweise gegen einen im Blut stets vorhandenen Bestandteil, beispielsweise ein Glubolin oder Albumin gerichteter Antikörper, immobilisiert sein. Alternativ kann das Kontrollfeld derart ausgestaltet sein, dass es lediglich die Benetzung mit Probenflüssigkeit äußerlich erkennbar durch Ausgabe eines messbaren Bindungssignals 12 anzeigt.

In dem gezeigten Teststreifen 3 bzw. Teststäbchen 4 wird ein optisches Bindungssignal 12, ein Farbstreifen, erzeugt, wenn in einem Testfeld 7 ein Quorum Sensing Zielmolekül 9 das entsprechende Detektionsmolekül 8 spezifisch bindet bzw. die applizierte Probe das Kontrollfeld erreicht, das als farbiger Streifen sichtbar wird.

Auf welche Arten ein solches Bindungssignal 12 generiert werden kann, wird unter Bezugnahme auf die beispielhaften Ausführungsformen des erfindungsgemäßen Schnelltests und Verweis auf Fig. 2 nachfolgend näher erläutert.

Beim erfindungsgemäßen Schnelltest zum Nachweis einer Sepsis wird zunächst eine Probe 6, beispielsweise eine Blutprobe, Urinprobe, Sputumprobe, Lymphflüssigkeitsprobe oder eine sonstige Körperflüssigkeitsprobe, die auf das Vorliegen einer Sepsis hin versucht werden soll, mit wenigstens zwei Detektionsmolekülen 8, die ein unterschiedliches Quorum Sensing Zielmolekül 9 aus der Probe 6 spezifisch bindet, unter Bedingungen in Kontakt gebracht, die ein Binden des Zielmolekül 9 an die Detektionsmoleküle 8 ermöglichen. In dem in Fig. 2 gezeigten Ablaufschema, das in der Ecke unten links beginnt, ist das Detektionsmolekül 8 beispielhaft als Antikörper dargestellt, der über ein Verknüpfungsmolekül 13 (auch Linker genannt) auf einen Träger 2, beispielsweise einem Biochip 14 oder einem Mikroarraysubstrat 15 immobilisiert ist. Nach dem in Kontaktbringen wird beim erfindungsgemäßen Schnelltest überprüft, ob Zielmoleküle 8 an die wenigstens zwei Detektionsmoleküle 9 gebunden haben.

In Fig. 2 sind beispielhaft drei mögliche Varianten, die Bindung eines Zielmoleküls 9 an ein Detektionsmolekül 8 zu überprüfen, dargestellt: der direkte Nachweis mit einem Nachweisreagenz 16a (Alternative 2a), der kompetitive Nachweis mittels eines Nachweisreagenzes 16b (Alternative 2b) sowie ein sogenannter Sandwich-Assay (Alternative 2c).

Beim direkten Nachweis wird als Nachweisreagenz 16a, das ein messbares Bindungssignal 12 ausgibt, ein weiterer Antikörper zugegeben. Der als Nachweisreagenz 16a eingesetzte weitere Antikörper ist mit einem Marker 28 versehen, und in der Lage, das am Deteketionsmolekül 8 gebundene Zielmolekül 9 spezifisch zu binden. Die Bindung des Nachweisreagenzes 16a am Zielmolekül 9 zeigt ein vom Marker 28 des Nachweisreagenzes 16a erzeugtes Bindungssignal 12 an. Das Bindungssignal kann beispielsweise ein Farbsignal sein, wenn der Marker 28 eine fluoreszierende Verbindung oder ein Enzym ist, das eine Farbreaktion katalysiert.

Beim kompetitiven Assay wird als Nachweisreagenz 16a ein Analyt zugesetzt, der mit dem Zielmolekül 9 um den Bindungsplatz am Detektionsmolekül 8 konkurriert. Der das Nachweisreagenz 16b bildende Analyt ist ebenfalls mit einem Marker 28 versehen, der ein Bindungssignal ausgebbar ausgestaltet ist. Der kompetitive Assay hat den Vorteil, dass damit nicht nur qualitativ nachgewiesen werden kann, dass das Zielmolekül 9 in der Probe 6 enthalten ist, sondern auch eine quantitative Bestimmung der Menge an Zielmolekül 9 in der Probe abgegeben werden kann. Je mehr Zielmoleküle 9 in der Probe 6 enthalten sind, umso mehr Zielmoleküle 9 bleiben nach Zugabe des Nachweisreagenzes 16b am Detektionsmolekül 8 gebunden. Dies heißt, dass bei einer hohen Konzentration des Zielmoleküls 9 in der Probe 6 nur wenige Moleküle des Nachweisreagenzes 16b an die Detektionsmoleküle 8 binden und folglich ein schwaches Bindungssignal ausgegeben wird. Liegt dagegen nur wenig oder gar kein Zielmolekül 9 in der Probe 6 vor, werden fast alle bzw. sämtliche Detektionsmoleküle 8 von dem Nachweisreagenz 16b besetzt und es wird ein entsprechend starkes Bindungssignal 12 ausgegeben.

Eine weitere Option, das Binden eines Zielmoleküls 9 an ein Detektionsmolekül 8 zu überprüfen, ist der sogenannte Sandwich-Assay, der auch in herkömmlicher ELISA (Enzyme-linked Immunosorbent Assays)-Verfahren eingesetzt wird. Dabei wird in einem ersten Nachweisschritt zunächst ein sekundärer Antikörper 16c, der in der Lage, spezifisch an das am Detektionsmolekül 8 gebundene Zielmolekül 9 zu binden. Anschließend wird in einem zweiten Schritt das eigentliche Nachweisreagenz 16d zugegeben, bei dem es sich um einen mit einem Marker 28 versehenen weiteren Antikörper 16d handelt, der spezifisch den Sekundärantikörper 16c bindet. Die daraus resultierende Sandwich-Struktur, bei welcher Marker 28 des Nachweisreagenzes 16d ein messbares Bindungssignal 12 ausgibt, ist in Fig. 2 unten rechts dargestellt.

Die Reagenzien 16a-16d, die zur Angabe des messbaren Bindungssignals 12 führen, können bei dem Teststreifen 3 bzw. Teststäbchen 4 aus Fig. 1 beispielsweise in Flussrichtung vor dem jeweiligen Nachweisfeld 7 im Trägermaterial so platziert werden, dass die Probe 6 die Reagenzien 16a-16d mitnimmt und in die Nachweisfelder 7 transportiert.

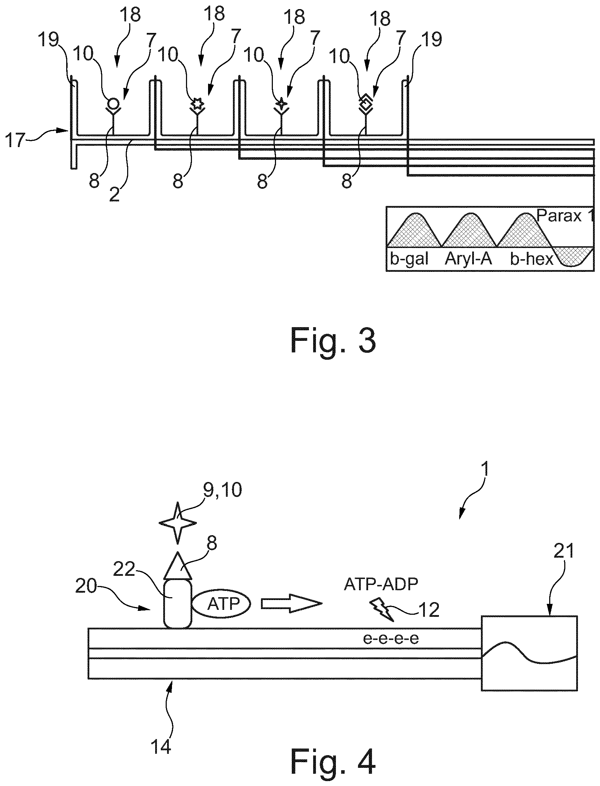

In Fig. 3 ist eine schematische Darstellung einer weiteren Vorrichtung 1 gezeigt. Im Folgenden wird lediglich auf die Unterschiede zur Vorrichtung 1 der vorherigen Figuren eingegangen. Für Elemente, deren Funktion und/oder Aufbau identisch zu Elementen der vorherigen Figuren ist, werden dieselben Bezugszeichen verwendet.

Bei der Vorrichtung 1, die in Fig. 3 gezeigt ist, wird als Träger 2 eine Mikrotiterplatte 17 verwendet. Die einzelne Vertiefungen 18 der Platte 17 enthalten jeweils ein Nachweisfeld 7.

In jedem Nachweisfeld 7 ist ein Detektionsmolekül 8 angeordnet, das ein spezifisches Quorum Sensing-assoziiertes Zielmolekül 10 bindet. In jedem Nachweisfeld 7 ist ein anderes Detektionsmolekül 8 angeordnet, so dass in jedem Nachweisfeld 7 ein unterschiedliches Quorum Sensing-assoziiertes Zielmolekül 10 nachgewiesen werden kann. Die Antikörper 8, welche die Detektionsmoleküle 8 bei der Vorrichtung 1 aus Fig. 3 darstellen, binden, von links nach rechts betrachtet, die folgenden Quorum Sensing-assoziierten Zielmoleküle 10: beta-Galaktosidase (dargestellt durch einen Kreis), beta-Hexosaminidase (dargestellt durch einen sechszackigen Stern), Arylsulphatase A (dargestellt durch einen vierzackigen Stern) und Paraxonase 1 (dargestellt durch eine Raute).

Im Unterschied zur vorherigen Vorrichtung sind die Detektionsmoleküle 8 in den Nachweisfeldern 7 bei der in Fig. 3 gezeigten Vorrichtung 1 weder direkt noch über einen Linker mit dem Träger 2 verbunden, sondern an eine auf der Innenwand der Vertiefungen 18 aufgebrachte Polymermatrix 19 gekoppelt. Durch die entsprechende Auswahl der Polymermatrix 19 kann, beispielsweise wenn diese über funktionelle Gruppen verfügt, auf einfache Weise eine hohe Anzahl an Detektionsmolekülen 8 in der jeweiligen Vertiefung 18 immobilisiert werden. Ferner gibt es eine Vielzahl an Polymeren, die biokompatibel sind und eine unspezifische Ablagerung von Zielmolekülen 9, 10 vermeiden.

In der in Fig. 3 gezeigten Vorrichtung stellen die Zielmoleküle 10 Quorum Sensing-assoziierte Enzyme dar, deren Aktivitäten beim Vorliegen einer Sepsis, im Vergleich zu gesunden Patienten, als direkte Folge einer erhöhten Konzentration an Quorum Sensing-Molekülen verändert ist. Die Veränderung der Quorum Sensing-assoziierten Zielenzyme 10 ist schematisch durch die Wellen in Fig. 3 unten rechts angedeutet. So ist bei einer Sepsis die Aktivität von dem Quorum Sensing-assoziierten Zielmolekülen beta-Galaktosidase, beta-Hexosaminidase und Arylsulphatase A erhöht, und die Enzymaktivität von Paraxonase 1 reduziert. Ob eine veränderte Enzymaktivität der Quorum Sensing-assoziierten Zielmoleküle 10 vorliegt, lässt sich beispielsweise durch die Bestimmung der Konzentration des jeweiligen Quorum Sensing-assoziierten Zielenzyms 10 in der Probe oder die Messung der jeweiligen Enzymaktivität ermitteln. Als Referenz kann beispielsweise eine definierte Schwellenaktivität bzw. Schwellenkonzentration zu Rate gezogen werden oder eine vergleichbare Probe eines nicht mit Sepsis infizierten gesunden Patienten vermessen werden.

Bei der in Fig. 3 dargestellten Vorrichtung 1 müssen die jeweiligen Detektionsmoleküle 8 in der entsprechenden Vertiefung 18 nicht am Träger 2 bzw. der Polymermatrix 19 immobilisiert sein. Die Detektionsmoleküle können auch in einer stabilen Darreichungsform, beispielsweise kristallisiert, in der Vertiefung 18 so platziert werden dass sie sich bei Zugabe der Probe 6 in funktionsfähiger Form in Lösung gehen. Es ist ferner möglich, die entsprechenden Detektionsmoleküle 8 mit der Probe 6 separat in die jeweilige Vertiefung zu geben und anschließend, nachdem Probe 6 und Detektionsmoleküle 8 in der Vertiefung 18 in Kontakt gebracht wurden, den erfindungsgemäßen Schnelltest durchzuführen.

Nachfolgend wird auf eine dritte Vorrichtung 1 unter Bezugnahme auf die Fig. 4 näher eingegangen.

In Fig. 4 ist das Detektionsmolekül 8 auf dem Signalwandler 20 immobilisiert. Der Signalwandler 20 wiederum steht mit einem Biochip 14, der einen Halbleiter aufweist, in Verbindung. Der Halbleiter nimmt, wie nachfolgend noch näher erläutert werden wird, bei Bindung eines Zielmoleküls 9, 10 am Detektionsmolekül 8 freie Elektronen auf und gibt einen Stromfluss als Bindungssignal 12 aus, der von einer Messvorrichtung 21, in der gezeigten Ausführungsform einer digitalen Messvorrichtung, angezeigt und quantifiziert werden kann.

In der Ausführungsform der Fig. 4 wird als Signalwandler 20 eine Histidin-Kinase 22 verwendet. Die Histidin-Kinase 20 ist an einer Stelle mit der Halbleiterplatte des Biochips 14 verbunden und andererseits mit dem Detektionsmolekül 8 gekoppelt. Die Kopplung bildet einen Komplex aus Detektionsmolekül 8 und Histidin-Kinase 22. Sobald sich ein Zielmolekül 9, 10 an dem Detektionsmolekül 8 anlagert, wird die Histidin-Kinase 22 des Komplexes aktiviert und führt eine Hydrolyse-Reaktion des in der Histidinkinase gebundenen ATP durch. Bei der Hydrolyse wird ein Phosphatrest aus dem ATP gewonnen, es entsteht ADP und wird Energie freigesetzt. Die bei der ATP Hydrolyse frei werdende Energie kann potentiometrisch mittels Energie-Carrier auf den Halbleiter übertragen werden, was wiederum als Bindungssignal 12 ausgegeben, und durch die Messvorrichtung 21 festgestellt werden kann. Die Stärke des Stromflusses, die mit der Reaktionsenergie korreliert, kann bei der in Fig. 4 gezeigten Vorrichtung nicht nur die qualitative Bindung von Quorum Sensing-Zielmolekülen 9 bzw. Quorum Sensing-assoziierten Zielmolekül 10 am Detektionsmolekül 8 nachweisen, sondern auch eine quantitative Aussage über die Menge des Zielmoleküls 9, 10 in der Probe treffen.

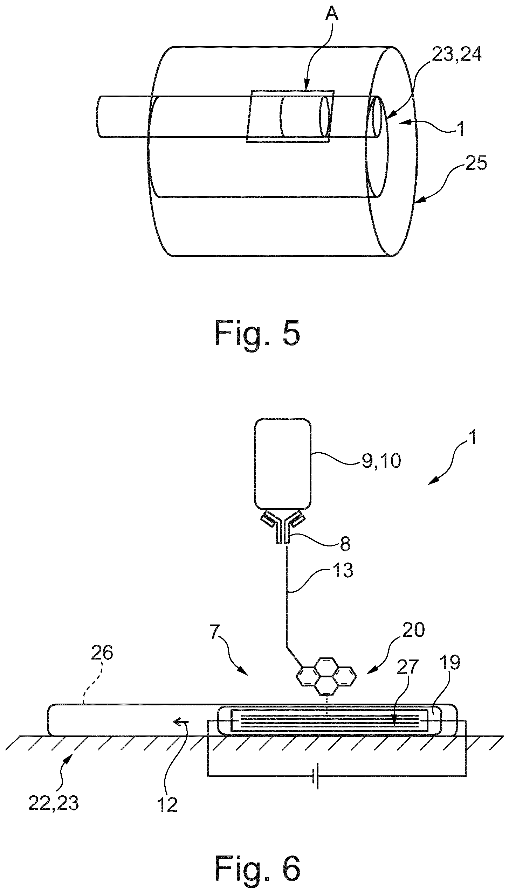

Im Folgenden wird unter Bezugnahme auf die Fig. 5 und 6 eine erfindungsgemäße Vorrichtung 1 gemäß einer weiteren Ausführungsform erläutert. Für Elemente, deren Funktion und/oder Aufbau identisch oder ähnlich zu Elementen der vorherigen Figuren ist, werden in den Fig. 5 und 6 dieselben Bezugszeichen verwendet. Bei dieser Ausführungsform stellt ein Katheter 23 bzw. eine Kanüle 24 der Träger 2 die erfindungsgemäße Vorrichtung 1 dar.

In Fig. 5 ist gezeigt, wie der Katheter 23 bzw. die Kanüle 24 in einem Lumen 25, das beispielhaft dargestellt ist und beispielsweise ein Blutgefäß sein kann, platziert ist. Die Zeichnungen geben allgemein die Größenangaben von Katheter 1, Lumen 3 sowie der Vorrichtung 1 lediglich schematisch und nicht maßstabsgetreu dar.

Die Vorrichtung 1 aus Fig. 5, deren Ausschnitt A im Detail in Fig. 6 schematisch dargestellt ist, umfasst einen elektrischen Leiter als Signalleiter 26, einen mit einem Polymer 19 beschichteten Signalwandler 20 und ein Nachweisfeld 7, das Antikörper als Detektionsmoleküle 8 umfasst. Der Antikörper 13 ist nicht direkt an das Polymer 19 gebunden, sondern über ein Verknüpfungsmolekül 13 gekoppelt. Als Linker werden kleine Moleküle bezeichnet, die z.B. zwei gleiche (homobifunktionell) oder zwei unterschiedliche (heterobifunktionell) funktionelle Gruppen haben. Ebenso ist die Länge des Linkers für die Funktion relevant. Zero-length crosslinker werden für eine Verbindung von zwei Molekülen ohne einen Platzhalter (Spacer) verwendet. Die Verwendung eines Linkers kann sich, insbesondere bei komplexen Molekülen wie Enzymen oder Antikörpern, fördernd auf die biologische Aktivität der immobilisierten Struktur auswirken. Das aktive Zentrum oder die aktive Domäne des Moleküls wird durch den Linker weiter weg von der Stammstruktur gebracht, an die das Molekül immobilisiert ist. Damit verringert sich die Gefahr einer Inaktivierung durch die Immobilisation. Eine andere Möglichkeit ist es, den Linker so zu wählen dass dieser nur an eine bestimmte Struktur im Zielmolekül bindet und somit den aktiven Bereich des Moleküls intakt lässt. Für die Kopplung eines IgG Antikörpers an Carboxylgruppen im Polymer kann man den zero-length Crosslinker EDC (1-Ethyl-3-[3-dimethylaminopropyl]carbodiimide hydrochloride) verwenden, welcher die Bildung einer Peptidbindung zwischen einer primären Aminogruppe im Antikörper und einer Carboxylgruppe des Polymers katalysiert.

Der Signalwandler 20 umfasst in der in Fig. 6 gezeigten Ausführungsform eine Elektrodenanordnung 27, welche eine Änderung des Widerstandes misst. Die Widerstandsänderung wird durch die Bindung des Zielmoleküles 9, 10 und einer damit verbundenen strukturellen Änderung des Verknüpfungsmoleküls 13 hervorgerufen, von dem Signalwandler 20 als Bindungssignal 12 ausgegeben und über den Signalleiter 26 nach außen transportiert.

Die Bindung des Zielmoleküls 9, 10 kann beispielsweise ein elektrisches Signal hinterlassen, wenn Bindungspartner eine elektroaktive Substanz ist und durch z. B. Massenzunahme aufgrund der Bindung seine Leitfähigkeit verändert. Im Falle von einem Schnelltest für Quorum-Sensing assoziierte Enzyme als Zielmoleküle 10 kann die Signalausgabe potentiometrisch erfolgen, weil dort Ladungsträger durch den enzymatischen Umsatz frei werden und diese die Stromflussänderung als Messwert ausgeben. Optisch kann die Bindung colometrisch detektiert, oder nachgewiesen werden, indem die Bindung die Lichtreflexions/-brechungseigenschaften ändert, was Lichtteilchen-Sensoren feststellen können.

### Bezugszeichen

- 1: Vorrichtung
- 2: Träger
- 3: Teststreifen
- 4: Teststäbchen
- 5: Applikationsfeld
- 6: Probe
- 7: Nachweisfeld
- 8: Detektionsmolekül
- 9: Quorum Sensing Zielmolelül
- 10: Quorum Sensing-assoziierte Zielmolelül
- 11: Kontrollfeld
- 12: Bindungssignal
- 13: Linker
- 14: Biochip
- 15: Mikroarraysubstrat
- 16a, 16b, 16d: Nachweisreagenz
- 16c: Sekundärer Antikörper
- 17: Mikrotiterplatte
- 18: Vertiefung
- 19: Polymermatrix
- 20: Signalwandler
- 21: Messvorrichtung
- 22: Histidin-Kinase
- 23: Katheter
- 24: Kanüle
- 25: Lumen
- 26: Signalleiter
- 27: Elektrodenanordnung
- 28: Marker

- A: Ausschnitt in Fig. 5

## Patentansprüche

1. Vorrichtung (1) zur Durchführung eines Sepsistestes, insbesondere in Form eines Schnelltestverfahrens, die wenigstens zwei Nachweisfelder (7) mit Detektionsmolekülen (8), die ein Quorum Sensing-Zielmolekül (9) spezifisch binden, umfasst, wobei das Quorum Sensing Zielmolekül (9) ein Homoserinlacton oder ein Autoinduktor-Peptid ist, und wobei die Detektionsmoleküle (8) in einem Nachweisfeld (7) ein Homoserinlacton und in einem anderen Nachweisfeld (7) ein Autoinduktor-Peptid spezifisch binden.

2. Vorrichtung (1) gemäß Anspruch 1, **dadurch gekennzeichnet, dass** die Detektionsmoleküle (8) ein Antikörper, ein spezifisch bindendes Fragment von einem Antikörper, ein Antigen, ein Peptid, ein Protein, eine Nukleinsäure, ein Aptamer, ein Ligand, ein Rezeptor, ein Hapten, ein Enzym, ein Enzyminhibitor, ein Enzymsubstrat oder ein Cofaktor von einem Enzym sind.

3. Vorrichtung (1) gemäß Anspruch 1 oder 2, **dadurch gekennzeichnet, dass** die Detektionsmoleküle (8) in den wenigstens zwei Nachweisfeldern (7) spezifisch binden mit einem der folgenden Quorum Sensing Zielmoleküle (9):
- N-(11-carboxy-3-oxoundecanoyl)-L-homoserinlacton, N-(5-carboxypentanoyl)-L-homoserinlacton, N-(11-carboxy-3-hydroxyundecanoyl)-L-homoserinlacton, oder N-(9-carboxynonanoyl)-L-homoserinlacton, oder
- Furanosyl-borat-diester.

4. Vorrichtung (1) gemäß einem der Ansprüche 1 bis 3, **dadurch gekennzeichnet, dass** die Detektionsmoleküle (8) direkt oder indirekt über ein Verknüpfungsmolekül (13) und/oder eine Polymermatrix (19) auf einem Träger (2) immobilisiert sind.

5. Vorrichtung (1) gemäß Anspruch 4, **dadurch gekennzeichnet, dass** der Träger (2) ein Teststreifen (3), ein Teststäbchen (4), eine Mikrotiterplatte (17), ein Biochip (14), ein Mikroarraysubstrat (15), ein Katheter (23), eine Kanüle (24) oder ein Signalwandler (20) ist.

6. Vorrichtung (1) gemäß einem der Ansprüche 1 bis 5, **gekennzeichnet durch** eine Messvorrichtung (21) zur Detektion, vorzugsweise zur Detektion und Quantifizierung der Bindung zwischen Detektionsmolekül (8) und Zielmolekül (9, 10) in wenigstens einem, vorzugsweise in jedem Nachweisfeld (7).

7. Kit zur Durchführung eines Sepsis-Testes, insbesondere in Form eines Schnelltestverfahrens, umfassend wenigstens zwei Detektionsmoleküle (8), die ein unterschiedliches Quorum Sensing-Zielmolekül (9) spezifisch binden, wobei die unterschiedlichen Quorum Sensing Zielmoleküle ein Homoserinlacton und ein Autoinduktor-Peptid sind, sowie ferner umfassend wenigstens einen der folgenden Teile:
- ein Puffer,
- ein Waschreagenz,
- ein Nachweisreagenz zum Ausgeben eines messbaren Signals,
wobei die Detektionsmoleküle (8) im Kit vorzugsweise als eine Vorrichtung (1) gemäß einem der Ansprüche 1 bis 6 bereitgestellt werden.

8. Schnelltestverfahren zur Unterstützung der Diagnose einer Sepsis, umfassend die Schritte:
- in Kontaktbringen einer Probe (6) und wenigstens zweier Detektionsmoleküle (8), die ein unterschiedliches Quorum Sensing-Zielmolekül (9) binden, unter Bedingungen, die ein Binden der Zielmoleküle (9, 10) an die wenigstens zwei Detektionsmoleküle (8) ermöglichen, wobei die unterschiedlichen Quorum Sensing Zielmoleküle (9) ein Homoserinlacton und ein Autoinduktor-Peptid sind; und
- Überprüfen, ob Zielmoleküle (9, 10) an die wenigstens zwei Detektionsmoleküle (8) gebunden haben.

9. Schnelltestverfahren gemäß Anspruch 8, **dadurch gekennzeichnet, dass** die Probe (6) mit einer Vorrichtung (1) gemäß einem der Ansprüche 1 bis 7 in Kontakt gebracht wird.

10. Schnelltestverfahren gemäß Anspruch 8 oder 9, **dadurch gekennzeichnet, dass** die Probe (6) eine Blutprobe, eine Urinprobe, eine Sputumprobe, eine Lymphflüssigkeitsprobe oder eine sonstige Körperflüssigkeitsprobe ist.

11. Schnelltestverfahren gemäß einem der Ansprüche 8 bis 10, **dadurch gekennzeichnet, dass** ein Nachweisreagenz (16a, 16b, 16d), das ein messbares Bindungssignal (12) ausgibt, zum Überprüfen des Bindens zugegeben wird.

12. Schnelltestverfahren gemäß Anspruch 11, **dadurch gekennzeichnet, dass** das messbare Bindungssignal (12) quantifiziert wird.

13. Schnelltestverfahren gemäß einem der Ansprüche 8 bis 12, **dadurch gekennzeichnet, dass** in Abhängigkeit vom Zielmolekül (9, 10), das spezifisch mit einem Detektionsmolekül (8) bindet, der Sepsis-Erreger charakterisiert, vorzugsweise der Gram-Status des Sepsis-Erregers bestimmt wird.

## Claims

1. Device (1) for implementation of a sepsis test, particularly in the form of a rapid test, which device (1) comprises at least two detection fields (7) with detection molecules (8) that specifically bind a quorum sensing target molecule (9), wherein the quorum sensing target molecule (9) is a homoserine lactone or an auto-inductor peptide, and whereby the detection molecules (8) in one detection field (7) bind a homoserine lactone and the detection molecules (8) in another detection field (7) specifically bind an auto-inductor peptide.

2. Device (1) according to claim 1, **characterized in that** the at least one detection molecule (8) is an antibody, a fragment of an antibody that is specifically binding, an antigen, a peptide, a protein, a nucleic acid, an aptamer, a ligand, a receptor, a hapten, an enzyme, an enzyme inhibitor, an enzyme substrate or a co-factor of an enzyme.

3. Device (1) according to claim 1 or 2, **characterized in that** the detection molecules (8) in the at least two detection fields (7) specifically bind with one of the following quorum sensing target molecules (9):
- N-(11-carboxy-3-oxoundecanoyl)-L-homoserine lactone, N-(5-carboxypentanoyl)-L-homoserine lactone, N-(11-carboxy-3-hydroxyundecanoyl)-L-homoserine lactone, N-(9-carboxynonanoyl)-L-homoserine lactone, or
- a furanosyl borate diester.

4. Device (1) according to any one of claims 1 to 3, **characterized in that** the detection molecules (8) are immobilized on a carrier (2) directly or indirectly through a linking molecule (13) and/or a polymer matrix (19).

5. Device (1) according to claim 4, **characterized in that** the carrier (2) is a test strip (3), a test stick (4), a micro-titer plate (17), a biochip (14), a micro-array substrate (15), a catheter (23), a cannula (24) or a signal converter (20).

6. Device (1) according to any one of claims 1 to 5, **characterized by** a measurement unit (21) for detection, preferably for the detection and quantification of the binding between the detection molecule (8) and the target molecule (9, 10) in at least one, preferably in each detection field (7).

7. Kit for the implementation of a sepsis test, particularly in form of a rapid test, comprising at least two detection molecules (8) that specifically bind a different quorum sensing target molecule (9), whereby the different quorum sensing target molecules (9) are a homoserine lactone and an auto-inductor peptide, as well as further comprising at least one of the following elements:
- a buffer,
- a washing reagent,
- a detection reagent to emit a measurable signal,
whereby the detection molecules (8) in the kit are preferably provided as a device (1) according to any one of claims 1 to 6.

8. Rapid test for supporting the diagnosis of sepsis, comprising the following steps:
- bringing a sample (6) into contact with at least two detection molecules (8) that specifically bind a different quorum sensing target molecule (9) under conditions that enable binding of the target molecule (9, 10) with the at least two detection molecules (8), whereby the different quorum sensing target molecules (9) are a homoserine lactone and an auto-inductor peptide; and
- verifying whether a target molecule (9, 10) has bonded with the at least two detection molecules (8).

9. Rapid test according to claim 8, **characterized in that** the sample (6) is brought in contact with a device (1) according to any one of claims 1 to 7.

10. Rapid test according to claim 8 or 9, **characterized in that** the sample (6) is a blood sample, a urine sample, a sputum sample, a lymphatic fluid sample or another body fluid sample.

11. Rapid test according to any one of claims 8 to 10, **characterized in that** a detection reagent (16a, 16b, 16d) that emits a measurable binding signal (12) is added for verifying the binding process.

12. Rapid test according to claim 11, **characterized in that** the measurable binding signal (12) is quantified.

13. Rapid test according to any one of claims 8 to 12, **characterized in that** the sepsis pathogen is characterized, preferably **in that** the gram status of the sepsis pathogen is determined, as a function of the target molecule (9, 10) that specifically binds with a detection molecule (8).

## Revendications

1. Dispositif (1) permettant de réaliser un test de sepsis, notamment sous forme d'un procédé rapide, qui comprend au moins deux champs révélateurs (7) avec des molécules de détection (8) qui se lient spécifiquement à une molécule cible de quorum-sensing (9), dans lequel la molécule cible de quorum-sensing (9) est une homosérine lactone ou un peptide auto-inducteur, et dans lequel les molécules de détection (8) se lient spécifiquement à une homosérine lactone dans un champ révélateur (7) et à un peptide auto-inducteur dans un autre champ révélateur (7).

2. Dispositif (1) selon la revendication 1, **caractérisé en ce que** les molécules de détection (8) sont un anticorps, un fragment d'anticorps à liaison spécifique, un antigène, un peptide, une protéine, un acide nucléique, un aptamère, un ligand, un récepteur, un haptène, une enzyme, un inhibiteur enzymatique, un substrat enzymatique ou un cofacteur d'une enzyme.

3. Dispositif (1) selon la revendication 1 ou 2, **caractérisé en ce que** les molécules de détection (8) dans les au moins deux champs révélateurs (7) se lient spécifiquement à l'une des molécules cibles de quorum-sensing (9) suivantes :
- la N-(11-carboxy-3-oxoundécanoyl) L-homosérine lactone, la N-(5-carboxypentanoyl) L-homosérine lactone, la N-(11-carboxy-3-hydroxyundécanoyl) L-homosérine lactone ou la N-(9-carboxynonanoyl) L-homosérine lactone, ou
- le furanosyl borate diester.

4. Dispositif (1) selon l'une des revendications 1 à 3, **caractérisé en ce que** les molécules de détection (8) sont immobilisées, de manière directe ou indirecte, sur un support (2) par le biais d'une molécule de liaison (13) et/ou d'une matrice polymère (19).

5. Dispositif (1) selon la revendication 4, **caractérisé en ce que** le support (2) est une bandelette réactive (3), un bâtonnet réactif (4), une plaque de microtitrage (17), une biopuce (14), un substrat de micromatrice (15), un cathéter (23), une canule (23) ou un transducteur (20).

6. Dispositif (1) selon l'une des revendications 1 à 5, **caractérisé par** un dispositif de mesure (21) permettant la détection, de préférence la détection et la quantification de la liaison entre molécule de détection (8) et molécule cible (9, 10), dans au moins un, de préférence dans chaque champ révélateur (7).

7. Trousse pour la réalisation d'un test de sepsis, notamment sous forme de procédé rapide, comprenant au moins deux molécules de détection (8) qui se lient spécifiquement à une molécule cible de quorum-sensing (9) différente, dans lequel les molécules cibles de quorum-sensing différentes sont une homosérine lactone et un peptide auto-inducteur, et comprenant, en outre, au moins un des éléments suivants :
- un tampon,
- un réactif de lavage,
- un réactif de dépistage destiné à émettre un signal mesurable,
dans lequel les molécules de détection (8) de la trousse sont présentées de préférence en tant que dispositif (1) selon l'une des revendications 1 à 6.

8. Procédé de test rapide destiné à aider au diagnostic d'un sepsis, comprenant ces étapes :
- mise en contact d'un échantillon (6) et d'au moins deux molécules de détection (8) qui se lient à une molécule cible de quorum-sensing (9) différente dans des conditions qui permettent une liaison des molécules cibles (9, 10) aux au moins deux molécules de détection (8), dans lequel les molécules cibles de quorum-sensing (9) différentes sont une homosérine lactone et un peptide auto-inducteur ; et
- contrôle pour vérifier si les molécules cibles (9, 10) se sont liées aux au moins deux molécules de détection (8).

9. Procédé de test rapide selon la revendication 8, **caractérisé en ce que** l'échantillon (6) est mis en contact avec un dispositif (1) selon l'une des revendications 1 à 7.

10. Procédé de test rapide selon la revendication 8 ou 9, **caractérisé en ce que** l'échantillon (6) est un échantillon sanguin, un échantillon d'urine, un échantillon de crachats, un échantillon de liquide lymphatique ou un échantillon d'un autre liquide corporel.

11. Procédé de test rapide selon l'une des revendications 8 à 10, **caractérisé en ce qu'**un réactif de dépistage (16a, 16b, 16d) qui émet un signal de liaison mesurable (12) est ajouté pour vérifier la liaison.

12. Procédé de test rapide selon la revendication 11, **caractérisé en ce que** le signal de liaison mesurable (12) est quantifié.

13. Procédé de test rapide selon l'une des revendications 8 à 12, **caractérisé en ce que** l'on caractérise l'agent pathogène responsable d'un sepsis, de préférence on détermine le Gram de l'agent pathogène responsable d'un sepsis, en fonction de la molécule cible (9, 10) qui se lie spécifiquement à une molécule de détection (8).
